# EUROPÄISCHE PATENTSCHRIFT

(11) **EP 3 117 808 B1**
(45) Veröffentlichungstag und Bekanntmachung des Hinweises auf die Patenterteilung: **09.10.2019**
(21) Anmeldenummer: 16001349.6
(22) Anmeldetag: 15.06.2016
(51) Int. Cl.: A61F 13/08, A61H 9/00, A61F 5/01

(54) **VORRICHTUNG ZUR VERMEIDUNG EINER THROMBOSE IN DEN BEINVENEN**
DEVICE FOR PREVENTING A THROMBOSIS IN THE LEG VEINS
DISPOSITIF DESTINE A EVITER LA THROMBOSE DANS LES VEINES DES JAMBES

(30) Priorität: 15.06.2015 DE 202015004498 U
(43) Veröffentlichungstag der Anmeldung: 18.01.2017
(73) Patentinhaber: teveno-medi GmbH, 90522 Oberasbach (DE)
(72) Erfinder: Herrmann, Joachim, 68782 Brühl (DE); Paneutz, Willi, 90522 Oberasbach (DE)
(74) Vertreter: Küchler, Stefan

(56) Entgegenhaltungen:
- WO-A1-97/18788
- WO-A1-98/19638
- DE-A1-102011 114 461
- DE-U1-202013 101 111
- GB-A- 817 521
- US-A- 5 288 286

## Beschreibung

Die Erfindung richtet sich auf eine Vorrichtung zur Vermeidung einer Thrombose in den Beinvenen, umfassend wenigstens ein abgeschlossenes System mit zwei miteinander kommunizierenden, von einer flexiblen Hülle aus einem flexiblen, luftundurchlässigen Material umgebenen, mit einem strömungsfähigen Medium befüllten Kammern, nämlich wenigstens einer Kompressionskammer unterhalb der Fußsohle, wenigstens einer Expansionskammer im Bereich des Innenknöchels in unmittelbarem Kontakt mit dem dortigen Perforansvenensystem, die von einem das Fußgelenk umschließenden Halteband fest gegen einen Bereich zwischen dem Innenknöchel und der Achillessehne gepresst wird, und wenigstens einem Verbindungsbereich in Form eines Kammer- und/oder Schlauchabschnittes, der die Kompressionskammer mit der Expanisonskammer strömungstechnisch verbindet.

Um ein Absacken des Blutes in die Füße einer Person zu verhindern, sind u.a. bereits Kompressionsstrümpfe verwendet worden.

Im Stand der Technik ist ferner schon vorgeschlagen worden, durch Druck auf bestimmte Abschnitte des Unterschenkels, Fußgelenks oder -knöchels einzuwirken, um die Durchblutung des betreffenden Patienten zu fördern.

Hierzu kann ein System mit untereinander verbundenen Kammern aus einem flexiblen oder elastischen, aber fluidundurchlässigen oder gar luftundurchlässigen Material verwendet werden, welche unmittelbar auf die betreffende Person einwirken. Für solche flexiblen oder elastischen, aber fluidundurchlässigen oder gar luftundurchlässigen Materialien wird oftmals auf gummiartige Substanzen zurückgegriffen. Diese sind jedoch auf Dauer nicht hautverträglich und verursachen rasch Abschürfungen, wunde Bereiche, Entzündungen od. dgl.

Ein Beispiel dafür zeigt die DE 10 2011 114 461 A1. Diese beschreibt eine gattungsgemäße Vorrichtung zur Vermeidung einer Thrombose, insbesondere der tiefen Venenthrombose in den Beinen, umfassend wenigstens ein abgeschlossenes System mit zwei miteinander kommunizierenden, von einer flexiblen Hülle umgebenen, mit einem strömungsfähigen Medium befüllten Kammern, nämlich einer Kompressionskammer unterhalb der Fußsohle und einer Expansionskammer im Bereich des Innenknöchels, in unmittelbarem Kontakt mit dem dortigen Perforansvenensystem, so dass bei einer Komprimierung der an der Fußsohle angeordneten Kompressionskammer von der dann expandierenden, am Innenknöchel angeordneten Expansionskammer das Blut aus der dortigen Perforansvene in die tiefe Vene gepresst wird. Hier ist keine Vorkehrung zum Schutz des Anwenders vor Abschürfungen, Entzündungen od. dgl. getroffen. Hinzu kommt, dass sich bei einem inneren Überdruck nicht nur die Expansionskammer aufbläht, sondern auch die Strömungsverbindung zwischen Kompressions- und Expansionskammer, so dass dieser innerhalb eines Schuhs liegender Verbindungsbereich verstärkt zur Bildung von Abschürfungen od. dgl. beiträgt.

Aus den Nachteilen des beschriebenen Standes der Technik resultiert das die Erfindung initiierende Problem, eine gattungsgemäße Vorrichtung zur Vermeidung einer Thrombose derart weiterzubilden, dass diese bedenkenlos auch über längere Zeiträume hinweg getragen werden können, ohne Beeinträchtigung der betroffenen Hautabschnitte oder sonstige gesundheitliche Beeinträchtigungen.

Die Lösung dieses Problems gelingt dadurch, dass im Bereich der Expansionskammer und/oder der Kompressionskammer und/oder des jene verbindenden Kammer- und/oder Schlauchabschnitts die Hülle aus einem flexiblen, luftundurchlässigen Material ihrerseits zumindest an ihrer dem Fuß zugewandten Oberfläche von wenigstens einer Lage und/oder Hülle aus einem hautverträglichen, textilen Stoff oder Gewebe unmittelbar überdeckt oder umgeben ist, so dass in den davon überdeckten Bereichen ein flächiger Kontakt der Hülle aus einem flexiblen, luftundurchlässigen Material mit der Haut des Patienten vermieden ist, wobei die Hülle aus einem flexiblen, luftundurchlässigen Material aus zwei aufeinander liegenden und entlang des Randes lückenlos miteinander verschweißten Zuschnitten aus einer flexiblen Kunststofffolie gebildet ist, wobei in dem Verbindungsbereich zwischen der Kompressionskammer und der Expansionskammer zwischen den beiden aufeinander liegenden Zuschnitten der Kunststofffolie punktförmige Verschweißungen in kurzen gegenseitigen Abständen von 10 mm oder weniger existieren, welche jedoch in der Expansionskammer und in der Kompressionskammer fehlen, damit die Expansionskammer sich bei einem inneren Überdruck kissenförmig aufblähen kann, um ihren Innendruck an die Perforansvene weiterzugeben.

Insbesondere kann das abgeschlossene System, bestehend aus der Kompressionskammer, der Expansionskammer sowie ggf. einem jene verbindenden Kammer- und/oder Schlauchabschnitt, vollflächig von einem hautverträglichen, textilen Stoff oder Gewebe unmittelbar umgeben sein, so dass jeglicher flächiger Kontakt der flexiblen, luftundurchlässigen Hülle des abgeschlossenen Systems mit der Haut des Patienten vermieden ist.

Ein solcher, hautverträglicher Stoff- oder Gewebeabschnitt schiebt sich in den überdeckten Bereichen zwischen die Hülle aus einem luftundurchlässigen, aber nicht hautverträglichen Material und schützt damit die Haut der betreffenden Person an den betreffenden Stellen vor Reizungen oder gar Entzündungen.

Es hat sich als günstig erwiesen, dass die Expansionskammer sowie der jene mit der Kompressionskammer verbindende Kammer- und/oder Schlauchabschnitt von einem gemeinsamen Stoff- oder Gewebezuschnitt umgeben ist. Der im Allgemeinen gegenüber der Expansionskammer verjüngte Verbindungsabschnitt - der entweder unmittelbar zwischen die Kammern eingefügt und zusammen mit jenen hergestellt sein kann oder aus einem gesondert eingefügten Schlauchabschnitt bestehen kann - erlaubt es dem umgebenden Stoff- oder Gewebezuschnitt, sich in dem Übergangsbereich zwischen der Expansionskammer und dem Verbindungsabschnitt ebenfalls zu verjüngen. Diese Verjüngung dient einer zuverlässigen Verankerung des umhüllenden Stoff- oder Gewebezuschnitts, so dass jener von selbst nicht von der Expansionskammer herabrutschen kann, selbst wenn er wenigstens eine Öffnung zum Hindurchtritt des Verbindungsabschnitts aufweist.

Weitere Vorteile ergeben sich dadurch, dass der die Expansionskammer sowie den jene mit der Kompressionskammer verbindende Kammer- und/oder Schlauchabschnitt umgebende Stoff- oder Gewebezuschnitt entlang zweier Umfangsbereiche miteinander vernäht ist, so dass zwischen den beiden Nähten und einer durch Umbiegen des Zuschnitts gebildeten Abschnitt drei Öffnungen verbleiben. Im Allgemeinen haben die Expansionskammer und/oder die Kompressionskammer eine mehr flächige Gestalt, d.h., sie bestehen ähnlich wie ein Kissen aus zwei randseitig miteinander verbundenen flachen Zuschnitten. Bei Befüllung dehnen sie sich zwar im Bereich ihrer Mitte aus, während dabei die rundum laufenden Verbindungsnähte die beiden Zuschnitte zusammenhalten und für die Stabilität der Kissengestalt sorgen. Infolge dieser stabilen Kissengestalt bietet es sich an, zu deren Umhüllung einen flachen Zuschnitt zu verwenden, der einmal umgefaltet sein kann, um sich beiden Kissenhälften gleichermaßen anzuschmiegen. Wenn die dann aufeinander liegenden Zuschnitthälften entlang eines von der Umschlagfalte entfernten Umfangsabschnitts vernäht werden, so ergibt sich eine Tasche mit zwei Öffnungen. Bei zwei voneinander beabstandeten, randseitigen Nähten - jeweils im Abstand zu der Umschlagfalte - erhält man eine Tasche mit drei Öffnungen.

Die Erfindung lässt sich weiter perfektionieren, indem eine Öffnung des vernähten Stoff- oder Gewebezuschnitts zum Hindurchtritt des die Expansionskammer mit der Kompressionskammer verbindenden Kammer- und/oder Schlauchabschnitt dient. Auf der Kompressionskammer lastet beim Auftreten das ganze Körpergewicht. Aufgrund der dort herrschenden Kräfte ist dort eine stärkere Polsterung wünschenswert, wofür andere Materialien besser geeignet sind als die demgegenüber dünnere Umhüllung der Expansionskammer. Daher kann sich die Kompressionskammer außerhalb der Umhüllung für die Expansionskammer befinden.

Zwei Öffnungen des vernähten Stoff- oder Gewebezuschnitts können zum Hindurchtritt zweier Enden des das Fußgelenk umschließenden, die Expansionskammer gegen den Innenknöchel pressenden Haltebandes dienen. Unter einem stiegenden Innendruck bläht sich die Expansionskammer auf. Damit jedoch die daraus resultierende Verdickung des Kissens voll auf die Perforans-Vene geleitet wird, ist ein Halteband vorgesehen, welches das Fußgelenk umschließt und die Expansionskammer fest gegen die Perforansvene drückt - etwa auf Höhe des Knöchels, zwischen dem Innenknöchel und der Achillesferse.

Die Erfindung empfiehlt, dass ein die Kompressionskammer zumindest teilweise umgebender, hautverträglicher, textiler Stoff- oder Gewebeabschnitt aus Frottier gebildet ist. Frottier ist besonders weich und saugfähig, und polstert die Ferse gegenüber der Kompressionskammer besser ab.

Ein die Kompressionskammer zumindest teilweise, insbesondere an deren dem Fuß zugewandten Innenseite, umgebender, hautverträglicher, textiler Stoff- oder Gewebeabschnitt aus Frottier kann mit einem Strumpf vernäht sein, vorzugsweise mit dessen Innenseite, insbesondere mit dessen Fersenabschnitt. Insbesondere kann die Anordnung derart getroffen sein, dass ein die Kompressionskammer fußseitig umgebender Stoff- oder Gewebeabschnitt aus Frottier derart an die innenseite eines Strumpfs genäht ist, dass sich dazwischen eine Tasche zur Aufnahme der Kompressionskammer ergibt. Durch eine Öffnung in einer das Frottier-Gewebe mit dem Strumpf verbindenden Naht kann der Verbindungsabschnitt zur Expansionskammer aus einer solchen Tasche heraustreten.

Die Erfindung läst sich dahingehend weiterbilden, dass Bereiche der Sohle eines die erfindungsgemäße Vorrichtung aufnehmenden Strumpfs aus Frottier bestehen. Wenn insbesondere der Ballenbereich ebenfalls aus Frottier besteht, so ergibt sich einerseits eine einheitliche Haptik des Strumpfes, andererseits sind dann die das Körpergewicht tragenden Bereiche des Fußes gut gepolstert und können ggf. auch Schweiß gut aufnehmen.

Eine bevorzugte Konstruktionsvorschrift besagt, dass Bereiche eines die erfindungsgemäße Vorrichtung aufnehmenden Strumpfs mit aus einem anderen Material und/oder nach einem anderen Strickmuster hergestellt sind als andere, insbesondere damit der Strumpf bereichsweise eine höhere Elastizität aufweist. Frottier ist zwar ein dickes, weiches und saugfähiges Material, gleichzeitig aber wenig elastisch. In bestimmten Bereichen, bspw. im Bereich des Mittelfußes, ist aber eine erhöhte Dehnbarkeit wünschenswert, was durch Verwendung eines anderen Materials und/oder Strickmusters erreicht werden kann. Es hat sich als vorteilhaft erwiesen, zwischen einem dem Ballen oder Vorderfuß zugeordneten Bereich des Strumpfs und einem der Ferse zugeordneten Strumpfbereich einen beispielsweise ringförmigen Abschnitt des Strumpfes im Bereich des Mittelfußes aus einem anderen Material und/oder mit einem anderen Strickmuster herzustellen, um die erstgenannten Beeiche voneinander zu entkoppeln und dem Strumpf eine gewisse Dehnbarkeit zu erteilen, insbesondere auch im Hinblick auf eine optimale Passform.

Es liegt im Rahmen der Erfindung, dass ein die erfindungsgemäße Vorrichtung aufnehmender Strumpf im Bereich unterhalb seiner Öffnung mit einer Stulpe versehen ist, welche über ein um das Fußgelenk geschlungenes Halteband herabklappbar ist. Eine solche Anordnung erleichtert das Anziehen eines erfindungsgemäßen Strumpfs. Insbesondere kann zuerst der untere Teil des Strumpfs über den Fuß und die Ferse gezogen werden, dann wird der Beinabschnitt zuächst insgesamt hochgezogen, und nachdem ein die Expansionskammer an die Perforansvene drückendes Halteband geschlossen wurde, beispielsweise durch Verbinden von dessen Enden mittels daran vorgesehener, zueinander komplementärer Klettelemente, wird die äußere Lage des oberen strumpfabschnitts stulpenartig über das Halteband am Knöchel herabgeklappt, um jenes von außen unsichtbar zu verdecken.

Eine weitere Optimierung erfährt die Erfindung dadurch, dass ein die erfindungsgemäße Vorrichtung aufnehmender Strumpf im Bereich zwischen seiner Öffnung und einer angesetzten Stulpe eine weiche Beschaffenheit aufweist, insbesondere großmaschig gestrickt ist. Je nach Ausführungsform kann dieser Teil des Strumpfs dann nach Art einer Socke elastisch an die Wade heran oder nach Art eines Kniestrumpfs über jene nach oben gezogen werden.

Schließlich entspricht es der Lehre der Erfindung, dass die Expansionskammer zwischen dem Innenknöchel und der Achillessehne in unmittelbarem Kontakt mit dem dortigen Perforansvenensystem angeordnet ist, so dass bei einer Komprimierung der an der Fußsohle angeordneten Kompressionskammer von der dann expandierenden, am Innenknöchel angeordneten Expansionskammer das Blut aus der dortigen Perforansvene in die tiefe Vene gepresst wird. Dies ist der eigenntliche Zweck der Erfindung, die Unterstützung der Perforansvene beim Hochpumpen des venösen Blutes in Richtung zum Herzen. Um diese Aufgabe optimal erfüllen zu können, ist eine präzise Lage der Expansionskammer an der dafür vorgesehenen Stelle unmittelbar außerhalb der perforansvene wichtig. Die Expansionskammer wird dabei von dem Strumpf an drei Punkten gehalten und fixiert: Ein erster solcher Fixpunkt ist die Kompressionskammer, die in einer Tasche unterhalb der Ferse der betreffenden Person aufgenommen ist und ihre Position über den Verbindungsabschnitt der Expansionskammer mitteilt, insbesondere deren unterem Teil. Die beiden übrigen Fixpunkte befinden sich an dem den Fußknöchel umschließenden Halteband. Zu diesem Zweck kann das Halteband mit der Stoff- oder Gewebeumhüllung für die Expansionskammer verbunden sein, insbesondere vernäht, und/oder direkt mit der Expansionskammer, letzteres natürlich ohne deren Dichtheit zu beeinträchtigen, weil sich ansonsten kein genügender Druck aufbauen könnte. Besonders geeignet sind hierfür seitliche Ansätze an der Expansionskammer, insbesondere in Laufrichtung vor und hinter derselben, welche dann ggf. zusammen mit dem Halteband und/oder mit der Stoff- oder GewebeUmhüllung zusammengenäht sein können für eine dauerhafte, unlösbare Verbindung. Natürlich könnte stattdessen auch eine Klebeverbindung od. dgl. herangezogen werden. Bevorzugt ist die Verbindung derart gestaltet, dass die Oberkante des Haltebandes an der Oberkante der Expansionskammer entlang läuft. Somit befinden sich zwei der drei Fixpunkte im oberen Bereich der Expansionskammer, einmal im Bereich von deren vorderer, oberer Ecke oder Rundung, einmal im Bereich von deren hinterer, oberer Ecke oder Rundung.

Der dritte Fixpunkt liegt wie schon gesagt am unteren Ende der Expansionskammer, nämlich dort, wo jene in den Verbindungsabschnitt zu der Kompressionskammer übergeht. Zwischen diesen drei Fixpunkten ist die Expansionskammer quasi eingespannt und kann sich kaum verschieben, insbesondere auch dann, wenn das Halteband zusätzlich durch zwei schlitzförmige Öffnungen in dem Strumpf herausgeführt und also zum Öffnen und Schließen von außen erreichbar ist. Dazu gibt es idealerweise für jedes Ende des Haltebandes einen eigenen Schlitz in dem Strumpf. Diese schlitzförmigen Öffnungen in dem Strumpf sollten von je einer Naht eingefasst sein, damit sich dort nicht das Gewebe auftrennt.

Damit ist also die Expansionskammer über die drei Fixpunkte mit dem Strumpf verbunden und könnte sich allenfalls zusammen mit jenem verschieben. Dem kann jedoch durch einen rutschhemmenden Belag an der Innenseite des Strumpfs und/oder durch besonders elastisch gestaltete Bereiche desselben vorgebeugt werden. Zusätzlich kann nötigenfalls die Lage der Expansionskammer einfach durch Verschieben des Strumpfes wieder gerichtet werden. Dabei kommt der Erfindung die Tatsache zugute, dass sich der bevorzugte Druckpunkt auf der Perforansvene innerhalb einer natürlichen Mulde der Fußanatomie befindet, nämlich hinter dem Innenknöchel und vor der Achillessehne. Dort passt sich die kissenförmig prall aufgeblähte Expansionskammer bündig ein und zentriert sich quasi selbst, bzw. eine Fehlpositionierung wird von der betreffenden Person sofort als unangenehm empfunden und kann dann leicht korrigiert werden.

Bevorzugt weist das Kompressionskissen eine (teilweise) Füllung durch einen elastischen Körper auf, bspw. in Form eines Blockes aus einem weichen Schaumstoff, damit sich die Kompressionskammer bei Entlastung wieder von selbst in einem gewünschten Maß aufdehnt und aufbläht und dadurch Luft aus der Expansionskammer saugt.

Wenn - wie die Erfindung weiter vorsieht - die Expansionskammer keine solche Füllung aus einem elastischen Material aufweist, kann sich die Expansionskammer bei Aufdehnen der Kompressionskammer ungehindert zusammenziehen, im Idealfall sogar bis das verbleibende Restvolumen zu null wird oder vernachlässigbar gering ist. Dies hat den Vorteil, dass dann die Expansionskammer nicht auftträgt und also in einem Schuh nicht drückt.

In dem Verbindungsbereich zwischen der Expansionskammer und der Kompressionskammer existieren in kurzen gegenseitigen Abständen punktförmige Verschweißungen oder sonstige Punktverbindungen zwischen den beiden Lagen der Kunststofffolie so dass sich diese beiden Lagen kaum voneinander entfernen, selbst wenn ein innerer Überdruck herrscht. Infolgedessen verändert sich das Volumen innerhalb des Verbindungsbereichs selbst bei starken Druckschwankungen kaum.

Weitere Merkmale, Einzelheiten, Vorteile und Wirkungen auf der Basis der Erfindung ergeben sich aus den Unteransprüchen sowie aus der folgenden Beschreibung einer bevorzugten Ausführungsform der Erfindung sowie anhand der Zeichnung. Hierbei zeigt:
- Fig. 1: eine in einen Strumpf integrierte und an einem Bein getragene, erfindungsgemäße Vorrichtung mit einem abgeschlossenen System aus einem flexiblen oder elastischen, luftundurchlässigen Material zur Vermeidung einer Thrombose in den Beinvenen in einer perspektivischen Ansicht schräg von oben, wobei ein Strumpf für den rechten Fuß dargestellt ist;
- Fig. 2: eine der Anordnung aus Fig. 1 entsprechende Anordnung, ebenfalls in einen Strumpf integriert, schräg von unten gesehen, wobei der Strumpf umgestülpt ist, so dass man nun dessen Innenseite und seine innere Struktur erkennen kann, und wobei anstelle eines Strumpfs für den rechten Fuß der andere Strumpf des betreffenden Paars dargestellt ist, also der Strumpf für den linken Fuß;
- Fig. 3: das abgeschlossenes System aus einem flexiblen oder elastischen, luftundurchlässigen, aber nicht hautverträglichen Material der Vorrichtung nach Fig. 2, und eine jenes bereichsweise umgebende Hülle aus einem hautverträglichen Stoff- oder Gewebeabschnitt in einer Seitenansicht;
- Fig. 4: einen Längsschnitt durch die Fig. 3 parallel zur Papierebene, sowie
- Fig. 5: den ebenen Zuschnitt für die Hülle aus Fig. 3.

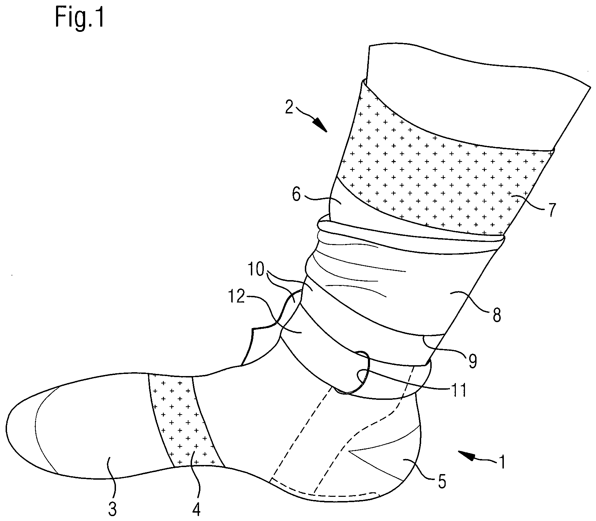

Die In Fig. 1 wiedergegebene Vorrichtung 1 zur Vermeidung einer Thrombose in den Beinvenen ist in einen Strumpf 2 integriert, der von einer Person in einem Schuh getragen werden kann.

Der Strumpf 2 verfügt über einen Vorderfußbereich 3 zur Aufnahme der Zehen und des Ballenbereichs eines Trägers, einen Mittelfußbereich 4 für den Mittelfuß des betreffenden Trägers, einen Fersenbereich 5, der sich der Ferse des Trägers anschmiegt, sowie einen Bein- oder Wadenbereich 6 für den Unterschenkel.

Der Vorderfußbereich 3 kann eine Sohle aus Frottier aufweisen, welche weich, stoßdämpfend und saugfähig ist; im oberen Abschnitt kann der Vorderfußbereich 3 aus einem üblichen Sockenmaterial bestehen.

Der Mittelfußbereich 4 kann demgegenüber als ringförmige Struktur aus einem elastisch dehnbaren Material und/oder mit einem die Elatizität erhöhenden Strickmuster ausgeführt sein, so dass sich insbesondere die Länge des Strumpfs 2, insbesondere aber auch dessen Umfang im Bereich des Mittelfußes, der Anatomie des betreffenden Trägers optimal anpassen kann. Der Bein- oder Wadenbereich 6 kann ebenfalls aus einem elastisch dehnbaren Material und/oder mit einem die Elastizität erhöhenden Strickmuster ausgeführt sein, so dass sich der Strumpf 2 genügend weit nach oben ziehen lässt, um am Unterschenkel der tragenden Person genügend Halt zu erfahren.

Insbesondere kann der obere Randbereich 7 des Bein- oder Wadenabschnitts 6 besonders elastich ausgebildet oder mit einem eingenähten Gummiband versehen sein, um dem Strumpf 2 besonderen Halt zu verleihen.

An der Außenseite des Bein- oder Wadenbereichs 6, vorzugsweise unterhalb des besonders elastischen Randbereichs 7, ist eine Stulpe 8 rundum laufend angenäht. Die Stulpe 8 hat die Struktur eines ringförmig geschlossenen Gewebestreifens, der mit einem seiner beiden endlosen Ränder 9 mit dem Bein- oder Wadenbereichs 6 verbunden ist, insbesondere vernäht. Die Aufgabe dieses Stulpenbereichs 8 ist es, beim Anziehen des Strumpfs 2 zunächst nach oben gezogen zu werden, wie der sonstige Bein- oder Wadenbereich 6 auch, nach Richten des Strumpfs 2 jedoch herabgeklappt werden kann, um einen zunächst noch sichtbaren Teil der Vorrichtung 1, nämlich einen Abschnitt 9 eines Haltebandes 10, der im Fersenbereich 5 zwischen zwei Schlitzen 11 in dem Strumpf 2 nach außen tritt, abzudecken. Im Idealfall erfolgt das Herabklappen natürlich faltenfrei; in Fig. 1 ist der Stulpenbereich 8 nur durch eine Raffung von dem übrigen Bein- oder Wadenbereich 6 abgehoben. Sobald die Stulpe 8 herabgeklappt ist, wird von dieser der zunächst noch sichtbare Bereich 9 des Haltebandes 10 vollständig verdeckt, und es ist von außen nicht erkennbar, dass sich in dem Fersenbereich 5 eine Einrichtung 1 zur Vermeidung einer Thrombose in den Beinvenen verbirgt. Dies erlaubt es auch Trägern und Trägerinnen von Sandalen oder niedrigen Schuhen, unerkannterweise eine solche Einrichtung 1 zu tragen.

Der Strumpf 2 ist vorzugsweise Bestandteil eines Strumpf-Paars, umfassend einen rechten Strumpf 2 und einen dazu spiegelbildlichen, linken Strumpf 2. Da - abgesehen von ihrer Spiegelsymmetrie - der linke Strumpf 2 völlig identisch zu dem rechten Strumpf 2 ist, soll im Folgenden zwischen linkem und rechtem Strumpf 2 nicht unterschieden werden.

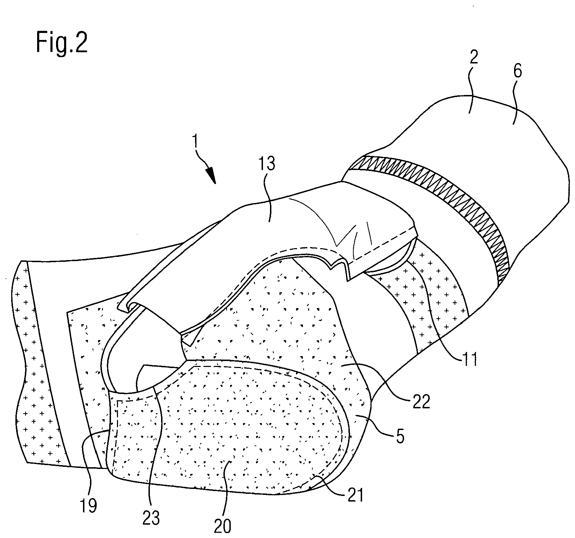

Um die Vorrichtung 1 zur Vermeidung einer Thrombose überhaupt zu erkennen, muss der Strumpf 2 umgestülpt werden, damit sein Inneres sichtbar wird. Dies ist in Fig. 2 erfolgt; allerdings wurde dort nicht der in Fig. 1 abgebildete, rechte Strumpf 2 umgestülpt, sondern der dazu spiegelbildlich aufgebaute, ansonsten jedoch identische, linke Strumpf 2, damit die Lage der einzelnen Bestandteile der Vorrichtugn 1 und des Strumpfs 2 an einander entsprechenden Stellen zu finden sind. Man kann sich beispielsweise vorstellen, dass der umgestülpte, linke Strumpf 2 in Fig. 2 auf einen Schuhleisten aufgezogen ist, so dass wieder die Fußform erkennbar ist; allerdings ist nun Innen und Außen vertauscht.

Man erkennt im Bein- und Wadenbereich 6 wieder einen der beiden Schlitze 11, durch welche das Halteband 10 nach außen tritt. Nun erkennt man aber nicht den üblicherweise außen liegenden Abschnitt 9 des Haltebandes, sondern nur einen kurzen, üblicherweise innen liegenden Abschnitt 12.

Mit diesem eigentlich innen liegenden Abschnitt 12 des Haltebandes 10 ist eine Hülle 13 verbunden, welche die Expansionskammer 14 sowie einen Verbindungsbereich 15 zwischen der Expansionskammer 14 und einer im Fersenbereich 5 vorzufindenden Kompressionskammer 16 strömungstechnisch verbindet.

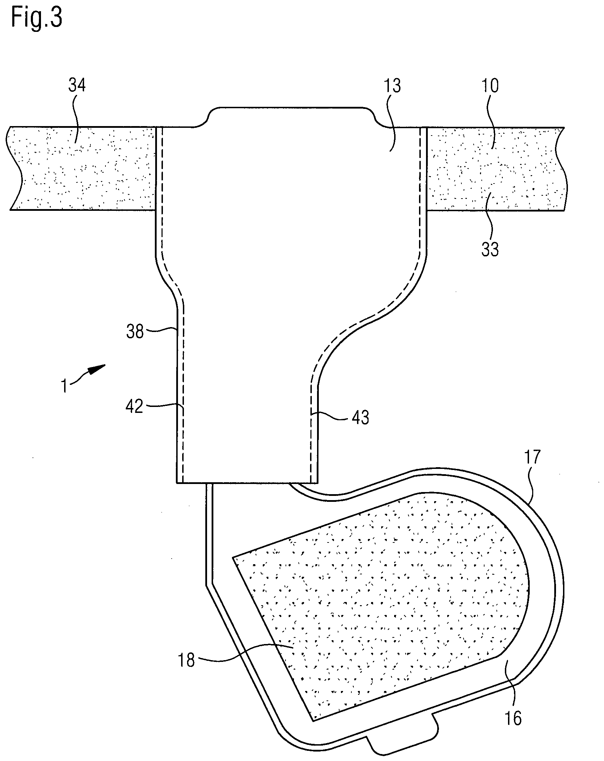

Diese Vorrichtung 1, bestehend aus der Kompressionskammer 16, der Expansionskammer 14, dem Verbindungsbereich 15, einer die beiden letzteren umgebenden Hülle 13 aus Stoff oder Gewebe, und dem mit der Hülle 13 und/oder der Expansionskammer 14 verbundenen Halteband 10, ist in Fig. 3 in aus dem Strumpf 2 herausgetrenntem und flach ausgebreiteten Zustand dargestellt.

Da die drei Kammern 14, 15, 16 aus zwei aufeinander liegenden und entlang des Randes lückenlos miteinander verschweißten Zuschnitten 17 aus einer flexiblen Kunststofffolie gebildet sind, haben die Kammern 14, 15, 16 in entleertem Zustand eine flächige Gestalt, welche sich in die Zeichenebene ausbreiten lässt.

Die Kompressionskammer 16 hat eine größere Fläche als die Expansionskammer 14. Im Betriebszustand ist das System 1 aus miteinander kommunizierenden Kammern 14, 15, 16 teilweise mit einem flüssigen oder gasförmigen Medium befüllt, beispielsweise mit Luft. Ferner kann innerhalb der Kompressionskammer 16 ein federndes Element 18 angeordnet sein, beispielsweise ein flacher, scheibenförmiger Block aus einem weichen Schaumstoff.

Die Kompressionskammer 16 hat vorzugsweise etwa die Grundfläche eines "D" und ist im Bereich des Strumpfes 2 im Bereich unterhalb der Ferse der betreffenden Person angeordnet, beispielsweise in eine dortige Tasche 19 eingeschoben, die an der Ober- und/oder Innenseite durch eine Lage 20 aus einem weichen, saufähigen Material wie Frottier gebildet ist. Diese Lage 20 ist vorzugsweise mittels einer rundum laufenden Naht 21 an der Innenseite 22 des Fersenbereichs 5 angenäht, allerdings mit einer Unterbrechung der Naht 21, so dass dort eine Öffnung 23 zum Einschieben der Kompressionskammer 16 und zum Hindurchtritt des Verbindungsbereichs 15 verbleibt. Vorzugsweise ist die Grundfläche der Tasche 19 hinsichtlich ihrer Form und Größe weitestgehend identisch mit der Grundfläche der Kompressionskammer 16, so dass jene möglichst exakt darin aufgenommen und fixiert ist. Die Öffnung 23 befindet sich vorzugsweise an der dem Innenknöchel zugewandten Seite des Strumpfs 2, in der Nähe der dortigen Vorderecke der Tasche 19.

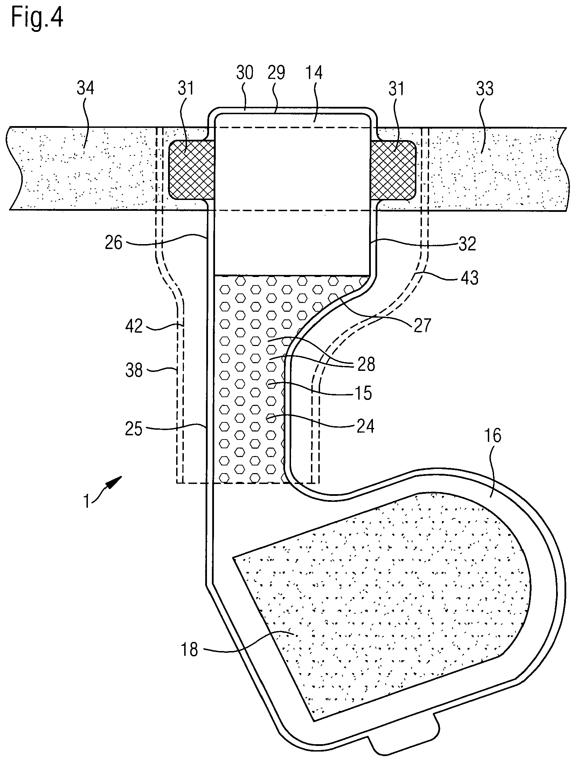

In Fig. 4 ist der innere Aufbau der Vorrichtung 1 zur Vermeidung einer Thrombose in den Beinvenen zu erkennen, da die Hülle 13 aus Stoff oder Gewebe dort aufgeschnitten ist. Man kann daher in Fig. 4 gut die drei Teile Expansionskammer 14, Verbindungskammer 15 und Kompressionskammer 16 unterscheiden.

Im Gegensatz zu der durch die Anatomie der Ferse bestimmten, etwa D-förmigen Grundfläche der Kompressionskammer 16 hat die Expansionskammer 14 eine rechteckige, sogar etwa quadratische Grundfläche. Die Grundfläche der Expansionskammer 14 entspricht nur etwa einem Viertel bis einem Drittel der Grundfläche der Kompressionskammer 16.

Der dazwischen liegende Verbindungsbereich 15 hat eine schmälere, längliche Gestalt mit einem Mittelbereich 24 von gleichbleibender Breite. Während die dem Vorderfuß zugewandte Vorderkante 25 des Mittelbereichs 24 mit der Vorderkante 26 der Expansionskammer 14 fluchtet, gibt es zwischen den beiden, der Achillessehne zugewandten Kanten einen geschwungenen Übergangsbereich 27.

Der Verbindungsbereich 15 unterscheidet sich von der Expansionskammer 14 dadurch, dass im Verbindungsbereich 15 in kurzen gegenseitigen Abständen punktförmige Verschweißungen 28 oder sonstige Punktverbindungen zwischen den beiden Lagen der Kunststofffolie existieren, so dass sich diese beiden Lagen kaum voneinander entfernen, selbst wenn ein innerer Überdruck herrscht. Infolgedessen verändert sich das Volumen des Verbindungsbereichs 15 selbst bei starken Druckschwankungen kaum.

Auch von der Kompressionskammer 16 ist der Verbindungsbereich 15 anhand der dort fehlenden punktförmigen Verschweißungen 28 zu unterscheiden. Während diese fehlenden Punktverschweißungen 28 in der Kompressionskammer 16 jedoch Platz für das dortige federnde Element 18 aus einem weichen Schaumstoff od dgl. schaffen soll, fehlen die Punktverschweißungen 28 in der Expansionskammer 14, damit diese sich bei einem inneren Überdruck kissenförmig aufblähen kann, um ihren Innendruck an die Perforansvene weiterzugeben.

Die Expansionskammer 14 ist an ihrer dem Fuß abgewandten Außenseite von dem Halteband 10 umgriffen, welches um das Fußgelenk geschlungen und geschlossen werden kann, um die Expansionskammer 14 fest gegen einen Bereich des Fußgelenks zwischen dem Innenknöchel und der Achillessehne zu pressen. Dabei verläuft das Halteband 10 parallel zu der der Kompressionskammer 16 abgewandten Oberkante 29 der Expansionskammer 14.

Um die Expansionskammer 14 überdies unverschieblich an dem Halteband 10 festzulegen, sind jenseits des randseitig umlaufenden Schweißbereichs 30 zwischen den beiden Lagen der das Kammersystem 14, 15, 16 bildenden Kunststofffolie Laschen 31 angeformt, welche durch vorzugsweise deckungsgleiche Ausstülpungen der beiden aufeinander gelegten Folienzuschnitte gebildet sind, und zwar nahe der Oberkante 29 der Expansionskammer 14, einmal an deren dem Vorderfuß zugewandten Vorderkante 26 und enmal an der der Achillessehne zugewandten, rückwärtigen Kante 32. Diese beiden Laschen 31 können beim Vernähen mit dem Halteband 10 bedenkenlos von einer Nähnadel durchstochen werden, da sie außerhalb der Expansionskammer 14 liegen und daher Stiche durch diese Laschen 31 die Dichtheit der Expansionskammer 14 nicht beeinträchtigen.

Wie man in Fig. 4 weiter erkennen kann, ist die Geometrie des Kammersystems 14, 15, 16 derart, dass die Symmetrieachse der D-förmigen Kompressionskammer 14 in eben ausgebreitetem Zustand zu der Oberkante 29 der Expansionskammer 14 nicht parallel, sondern konvergiert zu dem rückwärtigen Abschnitt 33 des Haltebandes 10, und entfernt sich gleichermaßen von dem vorderen Abschnitt 34 des Haltebandes 10 vor der Vorderkante 26 der Expansionskammer 14. Der Zwischenwinkel liegt etwa in der Größenordnung zwischen 15° und 45°, vorzugsweise zwischen 20° und 40°, insbesondere zwischen 25° und 35°, speziell bei etwa 30°. Der diesen Winkel verursachende Knick befindet sich im Kammersystem 14, 15, 16 zwischen der Kompressionskammer 16 und dem Verbindungsbereich 15.

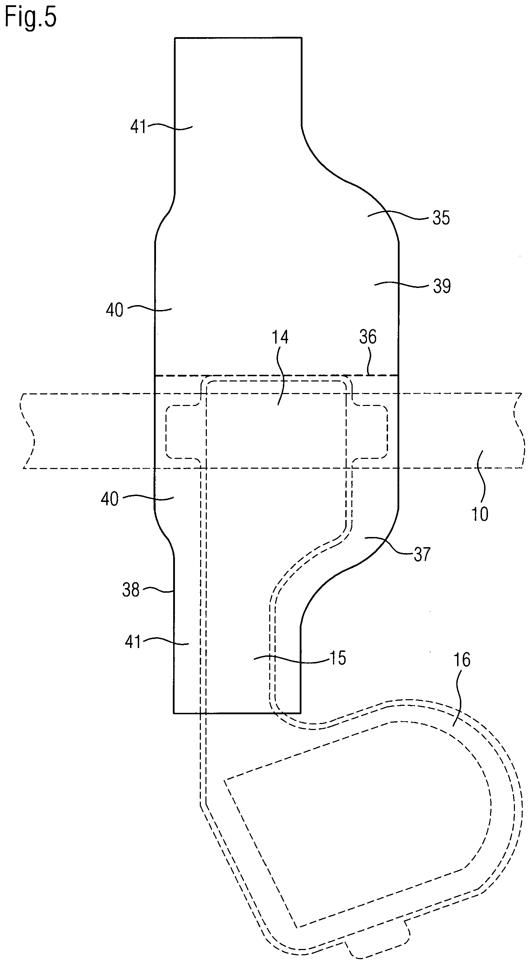

In Fig. 5 ist der Zuschnitt 35 für die Hülle 13 aus Stoff oder Gewebe zu sehen. Man erkennt, dass dieser Zuschnitt 35 symmetrisch zu der späteren Faltkante 36 ist, welche im fertigen Zustand entlang der Oberkante 29 der Expansionskammer 14 verläuft.

Diese Faltkante 36 ist etwa genauso lang wie oder etwas länger als der Abstand zwischen den Außenkanten der beiden Laschen 31 vor und hinter der Expansionskammer 14.

Die in Fig. 5 untere Hälfte 37 des Zuschnitts 35 liegt in Fig. 3 oberhalb der Expansions- und Verbindungskammern 14, 15 und verdeckt diese dadurch.

In Fig. 4 ist der Umriss 38 der unteren Hälfte 37 des Zuschnitts 35 gestrichelt wiedergegeben, und man erkennt daraus, dass dieser Umriss 38 dem randseitig umlaufenden Schweißbereich 30 zwischen den beiden Lagen der das Kammersystem 14, 15, 16 bildenden Kunststofffolie in einem Abstand folgt, der allerdings im Bereich der Expansionskammer 14 aufgrund der dortigen Laschen 31 breiter ist als auf Höhe des Verbindungsbereichs 15. Eine Hälfte 37 des Zuschnitts 35 entspricht also von ihrer Gestalt etwa dem Verlauf des randseitig umlaufenden Schweißbereichs 30 zwischen den beiden Lagen der das Kammersystem 14, 15, 16 bildenden Kunststofffolie im Bereich der Expansionskammer 14 und des Verbindungsbereichs 15; die andere Hälfte 39 ist spiegelbildlich dazu bezüglich der Faltkante 36.

Insbesondere erkennt man in Fig. 5 einen nahe der Faltkante 36 liegenden, breiteren Bereich 40 zur Aufnahme der Expansionskammer 14 und einen von der Faltkante 36 weiter entfernten, schmäleren Bereich 41 zur Aufnahme des Verbindungsbereichs 15.

In den Fig. 3 und 4 sind zwei seitliche Nähte 42, 43 angedeutet, einmal vor der Vorderkante 26 Expansionskammer 14, einmal hinter der rückwärtigen Kante 32 der Expansionskammer 14. Diese Nähte 42, 43 können sich von dem unteren bzw. von der Faltkante 36 am weitesten entfernten Ende der Hälften 37, 39 des Zuschnitts 35 jeweils bis unterhalb des Haltebandes 10 erstrecken, so dass dort Öffnungen zum Hindurchtritt des Haltebandes 10 verbleiben, oder die Nähte 42, 43 können sich durch das Halteband 10 hindurch bis zu der Faltkante 36 erstrecken. In letzterem Falle ist das Halteband 10 mit der Hülle 13 zu einer untrennbaren Einheit vernäht. Falls die Nähte 42, 43 dabei sogar durch die Laschen 31 vor und hinter der Expansionskammer 14 hindurch laufen, ist auch die Expansionskammer 14 mit dem Halteband 10 und der Hülle 13 unverschiebbar verbunden.

Dadurch erfährt die Expansionskammer 14 eine optimale Fixierung, einmal mittels der beiden Nähte 42, 43 an dem Halteband 10, zum anderen über den Verbindungsbereich 15 an der Kompressionskammer 16.

Da das Halteband 10 darüber hinaus durch die Schlitze 11 am Strumpf 2 geführt ist und außerdem durch Verbinden seiner endseitigen Klettelemente nach vorzugsweise einmaligem Umschlingen des Fußgelenks am Knöchel festgelegt ist, erfolgt eine absolute Fixierung der Expansionskammer 14 im Bereich ihrer Oberkante 29 am Fuß des Trägers, und zwar einmal am vorderen Ende der Expansionskammer 14 und einmal an deren hinterem Ende.

Ein dritter Fixierungspunkt ist durch die Kompressionskammer 16 gegeben, welche in die Tasche 19 eingesteckt und damit ebenfalls mit dem Strumpf 2 verbunden ist sowie über dessen Lage am Fuß bzw. in einem Schuh ebenfalls absolut festgelegt ist. Diese Fixierung wirkt auf das untere Ende der Expansionskammer 14 ein, dort wo der Verbindungsgebeich 15 in die Expansionskammer 14 mündet.

Da die Öffnung 23 der die Kompressionskammer 16 aufnehmende Tasche 19 des Strumpfs 2 zu dessen dem Fuß zugewandter Innenseite hin offen ist, liegt auch die damit über den Verbindungsbereich 15 kommunizierende Expansionskammer 14 innerhalb des Strumpfs; nur das Halteband 10 ist durch den Strumpf 2 nach außen geführt, insbesondere durch die beiden Schlitze 11 jeweils unmittelbar vor und hinter der Expansionskammer 14. Vorzugsweise sind die beiden Schlitze 11 des Strumpfs 2 zum Durchtritt der beiden Enden des Haltebandes 10 an ihren Rändern umgekettelt oder anderweitig abgenäht.

Die Expansionskammer 14 sowie der damit verbundene Abschnitt des Haltebandes 10 sowie der damit kommunizierende Verbindungsbereich 15 des Kammersystems 14, 15, 16 ist von der Hülle 13 aus Stoff oder Gewebe allseitig umgeben. Die Hülle 13 ist vorzugsweise einteilig hergestellt aus einem einzigen, spiegelsymmetrischen Zuschnitt 35. Da die Hülle 13 jeweils einen Teil der beiden Enden des Haltebandes 10 umgibt, also bis zu den beiden Schlitzen 11 in dem Strumpf 2 reicht und dort nach außen tritt, gelangt die Haut einer den Strumpf 2 tragenden Person weder irgendwo in Kontakt mit der Expansionskammer 14 noch mit dem Halteband 10, sondern ist stets durch die Hülle 13 aus einem hautverträglichen Stoff oder Gewebe davor geschützt. Da andererseits die vernähten Bereiche 42, 43 sich entlang des Verbindungsbereichs 15 bis unmittelbar an die Kompressionskammer 16 heran erstrecken, die wiederum in einer Tasche 19 aufgenommen und durch eine Frottierlage von der Haut der betreffenden Person getrennt ist, ist auch ein Hautkontakt mit dem Verbindungsbereich 15 und der Kompressionskammer 16 vermieden.

Die Hülle 13 aus einem Zuschnitt 35 eines hautverträglichen Stoffs oder Gewebes besteht vorzugsweise aus einem Baumwollgewebe, bevorzugt mit Polyamid- und/oder Elasthan-Anteilen.

Empfohlen wird ein Gewebe aus
- 80 % Baumwolle
- 18 % Polyamid
- 02 % Elasthan.

**Bezugszeichenliste**

| | | | |
|---|---|---|---|
| 1 | Vorrichtung | 26 | Vorderkante |
| 2 | Strumpf | 27 | Übergangsbereich |
| 3 | Vorderfußbereich | 28 | Punktverschweißung |
| 4 | Mittelfußbereich | 29 | Oberkante |
| 5 | Fersenbereich | 30 | Schweißbereich |
| 6 | Bein- oder Wadenbereich | 31 | Lasche |
| 7 | Randbereich | 32 | rückwärtige Kante |
| 8 | Stulpe | 33 | rückwärtiger Abschnitt |
| 9 | Rand | 34 | vorderer Abschnitt |
| 10 | Halteband | 35 | Zuschnitt |
| 11 | Schlitz | 36 | Faltkante |
| 12 | Haltebandabschnitt | 37 | eine Hälfte |
| 13 | Hülle | 38 | Umriss |
| 14 | Expansionskammer | 39 | andere Hälfte |
| 15 | Verbindungsbereich | 40 | breiterer Bereich |
| 16 | Kompressionskammer | 41 | schmälerer Bereich |
| 17 | Folienzuschnitt | 42 | Naht |
| 18 | Federndes Element | 43 | Naht |
| 19 | Tasche | | |
| 20 | Lage | | |
| 21 | Naht | | |
| 22 | Innnenseite | | |
| 23 | Öffnung | | |
| 24 | Mittelbereich | | |
| 25 | Vorderkante | | |

## Patentansprüche

1. Vorrichtung (1) zur Vermeidung einer Thrombose in den Beinvenen, umfassend wenigstens ein abgeschlossenes System mit zwei miteinander kommunizierenden, von einer flexiblen Hülle aus einem flexiblen, luftundurchlässigen Material (17) umgebenen, mit einem strömungsfähigen Medium befüllten Kammern, nämlich wenigstens einer Kompressionskammer (16) unterhalb der Fußsohle, wenigstens einer Expansionskammer (14) im Bereich des Innenknöchels in unmittelbarem Kontakt mit dem dortigen Perforansvenensystem, die von einem das Fußgelenk umschließenden Halteband (10) fest gegen einen Bereich zwischen dem Innenknöchel und der Achillessehne gepresst wird, und wenigstens einem Verbindungsbereich (15) in Form eines Kammer- und/oder Schlauchabschnittes, der die Kompressionskammer (16) mit der Expanisonskammer (14) strömungstechnisch verbindet, **dadurch gekennzeichnet, dass** im Bereich der Expansionskammer (14) und/oder der Kompressionskammer (16) und/oder des jene verbindenden Kammer- und/oder Schlauchabschnitts (15) die Hülle aus einem flexiblen, luftundurchlässigen Material (17) Ihrerseits zumindest an ihrer dem Fuß zugewandten Oberfläche von wenigstens einer Lage (20) oder Hülle (13) aus einem hautverträglichen, textilen Stoff oder Gewebe unmittelbar überdeckt ist, so dass in den davon überdeckten Bereichen ein flächiger Kontakt der Hülle aus einem flexiblen, luftundurchlässigen Material (17) mit der Haut des Patienten vermieden ist, wobei die Hülle aus einem flexiblen, luftundurchlässigen Material (17) aus zwei aufeinander liegenden und entlang des Randes lückenlos miteinander verschweißten Zuschnitten (17) aus einer flexiblen Kunststofffolie gebildet ist, wobei in dem Verbindungsbereich (15) zwischen der Kompressionskammer (16) und der Expansionskammer (14) zwischen den beiden aufeinander liegenden Zuschnitten (17) der Kunststofffolie punktförmige Verschweißungen in kurzen gegenseitigen Abständen von 10 mm oder weniger existieren, welche jedoch in der Expansionskammer (14) und in der Kompressionskammer (16) fehlen, damit die Expansionskammer (14) sich bei einem inneren Überdruck kissenförmig aufblähen kann, um ihren Innendruck an die Perforansvene weiterzugeben.

2. Vorrichtung (1) nach Anspruch 1, **dadurch gekennzeichnet, dass** die Expansionskammer (14) sowie der jene mit der Kompressionskammer (16) verbindende Kammer- und/oder Schlauchabschnitt (15) von einem gemeinsamen Stoff- oder Gewebezuschnitt (35) umgeben ist.

3. Vorrichtung (1) nach Anspruch 1 oder 2, **dadurch gekennzeichnet, dass** die die Expansionskammer (14) sowie den jene mit der Kompressionskammer (16) verbindenden Kammer- und/oder Schlauchabschnitt (15) umgebende Hülle (13) aus einem Stoff- oder Gewebezuschnitt (35) entlang zweier Umfangsbereiche (42,43) miteinander vernäht ist, so dass zwischen den beiden Nähten (42,43) und/oder zwischen jenen Nähten (42,43) und einem durch Umbiegen des Zuschnitts (35) gebildeten Abschnitt (36) insgesamt wenigstens eine Öffnung verbleibt.

4. Vorrichtung (1) nach einem der Ansprüche 1 bis 3, **dadurch gekennzeichnet, dass** eine Öffnung des zu einer Hülle (13) vernähten Stoff- oder Gewebezuschnitts (35) zum Hindurchtritt des die Expansionskammer (14) mit der Kompressionskammer (16) verbindenden Kammer- und/oder Schlauchabschnitts (15) dient.

5. Vorrichtung (1) nach einem der vorhergehenden Ansprüche, **dadurch gekennzeichnet, dass** zwei Öffnungen des zu einer Hülle (13) vernähten Stoff- oder Gewebezuschnitts (35) zum Hindurchtritt zweier Enden des das Fußgelenk umschließenden, die Expansionskammer (14) gegen den Innenknöchel pressenden Haltebandes (10) dienen, wobei diese Öffnungen auch von den betreffenden Nähten (42,43) unter Zusammennähen der Hülle (13) mit dem halteband (10) verschlossen sein können.

6. Vorrichtung (1) nach einem der vorhergehenden Ansprüche, **dadurch gekennzeichnet, dass** ein die Kompressionskammer (16) zumindest teilweise umgebender, hautverträglicher, textiler Stoff- oder Gewebeabschnitt aus Frottier gebildet ist.

7. Vorrichtung (1) nach einem der vorhergehenden Ansprüche, **dadurch gekennzeichnet, dass** die Expansionskammer (14) zwischen dem Innenknöchel und der Achillessehne in unmittelbarem Kontakt mit dem dortigen Perforansvenensystem angeordnet ist, so dass bei einer Komprimierung der an der Fußsohle angeordneten Kompressionskammer (16) von der dann expandierenden, am Innenknöchel angeordneten Expansionskammer (14) das Blut aus der dortigen Perforansvene in die tiefe Vene gepresst wird.

8. Vorrichtung (1) nach einem der vorhergehenden Ansprüche, **dadurch gekennzeichnet, dass** innerhalb der Kompressionskammer (16) ein elastischer Körper angeordnet ist, bspw. ein Block aus einem weichen Schaumstoff.

9. Vorrichtung (1) nach einem der vorhergehenden Ansprüche, **dadurch gekennzeichnet, dass** die Verschweißungen (28) zwischen den beiden Lagen der Kunststofffolie (17) in gegenseitigen Abständen von 5 mm oder weniger angeordnet sind.

10. Vorrichtung (1) nach einem der vorhergehenden Ansprüche, **dadurch gekennzeichnet, dass** sie innerhalb eines Strumpfs (2) angeordnet ist.

11. Vorrichtung (1) nach Anspruch 10, **dadurch gekennzeichnet, dass** ein die Kompressionskammer (16) zumindest teilweise umgebender, hautverträglicher, textiler Stoff- oder Gewebeabschnitt aus Frottier mit dem Strumpf (2) vernäht ist, vorzugsweise mit dessen Innenseite, insbesondere mit dessen Fersenabschnitt (5).

12. Vorrichtung (1) nach Anspruch 10 oder 11, **dadurch gekennzeichnet, dass** Bereiche der Sohle des die erfindungsgemäßen Vorrichtung (1) aufnehmenden Strumpfs (2) aus Frottier bestehen.

13. Vorrichtung (1) nach einem der Ansprüche 10 bis 12, **dadurch gekennzeichnet, dass** Bereiche des die erfindungsgemäßen Vorrichtung (1) aufnehmenden Strumpfs (2) mit einem anderen Strickmuster hergestellt sind als andere, insbesondere damit der Strumpf (2) bereichsweise eine höhere Elastizität aufweist.

14. Vorrichtung (1) nach einem der Ansprüche 10 bis 13, **dadurch gekennzeichnet, dass** der die erfindungsgemäße Vorrichtung (1) aufnehmende Strumpf (2) im Bereich unterhalb seiner Öffnung mit einer Stulpe (8) versehen ist, welche über ein um das Fußgelenk geschlungenes Halteband (10) herabklappbar ist.

15. Vorrichtung (1) nach einem der Ansprüche 10 bis 14, **dadurch gekennzeichnet, dass** der die erfindungsgemäße Vorrichtung (1) aufnehmende Strumpf (2) im Bereich zwischen seiner Öffnung und einer angesetzten Stulpe (8) eine weiche Beschaffenheit aufweist, insbesondere großmaschig gestrickt ist.

## Claims

1. Device (1) for preventing a thrombosis in the leg veins, comprising at least a closed system with two chambers communicating with each other, which are surrounded by a flexible envelope made of a flexible airtight material (17) and are filled with a flowable medium, namely at least one compression chamber (16) beneath the sole of the foot, at least one expansion chamber (14) in the area of the inner malleolus in direct contact with the perforator vein system there, which is pressed firmly against an area between the inner malleolus and the Achilles tendon by a retaining strap (10) encompassing the ankle, as well as at least a connecting area (15) in the shape of a chamber section and/or of a hose section, which connects the compression chamber (16) and the expansion chamber (16) in a fluidic way, **characterized in that**, in the area of the expansion chamber (14) and/or of the compression chamber (16) and/or of the chamber section and/or hose section (15) connecting the former, wherein the envelope made of a flexible airtight material (17) in turn is directly covered by at least one layer (20) or sleeve (13) made of a skin-compatible textile cloth or fabric at least at its surface facing the foot, so that in the areas covered thereby an areal contact between the envelope made of a flexible airtight material (17) and the skin of the patient is avoided, whereby the envelope made of a flexible airtight material (17) consists of two adjacent cuts (17) of a flexible plastic foil uninterruptedly welded to each other along their edges, wherein, in the connecting area (15) between the compression chamber (16) and the expansion chamber (14), there exist punctiform weldings between the two adjacent cuts (17) of the plastic foil in short mutual distances of 10 mm or less, which however are missing in the expansion chamber (14) and in the compression chamber (16), so that the expansion chamber (14) may blow up in the shape of a cushion due to an inner excess pressure in order to share its inner pressure with the perforator vein.

2. Device (1) according to claim 1, **characterized in that** the expansion chamber (14) as well as the chamber section or hose section (15) connecting the former to the compression chamber (16) are surrounded by a common cut (35) made of cloth or fabric.

3. Device (1) according to claim 1 or 2, **characterized in that** the sleeve (13) encompassing the expansion chamber (14) and the chamber section and/or the hose section (15) connecting the first with the compression chamber (16) is sewn from a cloth or fabric cut (35) along two peripheral areas (42,43), so that at least one opening remains between those two seams (42,43) and/or between those seams (42,43) and a section (35) formed by bending the cut (35) in all.

4. Device (1) according to one of claims 1 to 3, **characterized in that** an opening of the cloth or fabric cut (35) sewn to a sleeve (13) serves for the passage of the chamber section and/or hose section (15) connecting the expansion chamber (14) and the compression chamber (16).

5. Device (1) according to one of the preceding claims, **characterized in that** two openings of the cloth or fabric cut (35) sewn to a sleeve (13) serve for the passage of two ends of the retaining strap (10) encompassing the ankle and pressing the expansion chamber (14) against the inner malleolus, wherein these openings may also be closed by the regarding seams (42,43) upon sewing together the sleeve (13) with the retaining strap (10).

6. Device (1) according to one of the preceding claims, **characterized in that** a skin-compatible textile cloth or fabric cut (35) encompassing the compression chamber (16) at least partially is made of terry towelling.

7. Device (1) according to one of the preceding claims, **characterized in that** the expansion chamber (14) is arranged in direct contact with the perforating vein system between the inner malleolus and the Achilles tendon in such a way that, upon compression of the compression chamber (16) arranged at the sole of the foot, the blood is pressed from the perforating vein into the deep vein by the then expanding expansion chamber (14) arranged at the inner malleolus.

8. Device (1) according to one of the preceding claims, **characterized in that** an elastic body is arranged within the compression chamber (16), for example a block made of a soft foamed plastic.

9. Device (1) according to one of the preceding claims, **characterized in that** the weldings (28) between the two layers of the plastic foil (17) are arranged in mutual distances of 5 mm or less.

10. Device (1) according to one of the preceding claims, **characterized in that** it is arranged within a stocking (2).

11. Device (1) according to claim 10, **characterized in that** a skin-compatible textile cloth or fabric cut (35) made of terry towelling and encompassing the compression chamber (16) at least partially is sewn with the stocking (2), preferably with its inner side, especially with its heel section (5).

12. Device (1) according to claims 10 or 11, **characterized in that** areas of the sole of the stocking (2) accommodating the device (1) according to the invention consist of terry towelling.

13. Device (1) according to one of claims 10 to 12, **characterized in that** areas of the stocking (2) accommodating the device (1) according to the invention are manufactured with another knitting pattern than others, especially so that the stocking (2) shows a higher degree of elasticity in certain areas.

14. Device (1) according to one of claims 10 to 13, **characterized in that** the stocking (2) accommodating the device (1) according to the invention is provided in the area below its opening with a gauntlet (8) which is foldable down upon a retaining strap (10) wound around the ankle.

15. Device (1) according to one of claims 10 to 14, **characterized in that** the stocking (2) accommodating the device (1) according to the invention exhibits a soft consistency in the area between its opening and an attached gauntlet (8), especially is knitted with large meshes there.

## Revendications

1. Dispositif (1)pour éviter une thrombose dans les veines des jambes, comprenant au moins un système fermé présentant deux chambres communicantes l'une avec l'autre, entourées d'une enveloppe souple en un matériau souple imperméable à l'air (17), avec des chambres remplies d'un agent pouvant s'écouler, à savoir au moins une chambre de compression (16) sous la plante du pied, au moins une chambre de dilatation (14) au niveau de la malléole interne en contact direct avec le système de veines perforantes local, qui est comprimée de manière fixe par une bande de fixation (10) entourant la cheville contre une zone entre la malléole interne et le tendon d'Achille, et au moins une zone de liaison (15) sous la forme d'une section de chambre et/ou de flexible, qui relie par une technique d'écoulement la chambre de compression (16) à la chambre de dilatation (14), **caractérisé en ce que** dans la zone de la chambre de dilatation (14) et/ou de la chambre de compression (16) et/ou de la section de chambre et/ou de flexible (15)les reliant, l'enveloppe en un matériau souple, imperméable à l'air (17) est à son tour recouverte directement au moins sur sa surface orientée vers le pied, par au moins une couche (20) ou enveloppe (13) en un tissu ou une toiletextile tolérée par la peau de telle sorte que dans les zones recouvertes par celui-ci ou celle-ci, un contact surfacique de l'enveloppe en un matériau souple imperméable à l'air (17) et la peau du patient est évité, **en ce que** l'enveloppe en un matériau souple imperméable à l'air (17) est formée à partir de deux coupes (17) constituées d'un film plastique souple, soudées entre elles sans lacune, superposées et le long du bord, **en ce que** dans la zone de liaison (15) entre la chambre de compression (16) et la chambre de dilatation (14) entre les deux coupes (17) superposées du film plastique des soudures ponctuelles sont existantes à des intervalles rapprochés de 10 mm ou moins, lesquelles cependant manquent dans la chambre de dilatation (14) et dans la chambre de compression (16), afin que la chambre de dilatation (14) puisse se gonfler sous forme de coussin en cas de surpression interne pour transmettre sa pression interne à la veine perforante.

2. Dispositif (1) selon la revendication 1, **caractérisé en ce que** la chambre de dilatation (14) ainsi que la section de chambre et/ou de flexible (15) la reliant à la chambre de compression (16) est entourée par une coupe de tissu ou de toile (35) commune.

3. Dispositif (1) selon la revendication 1 ou 2, **caractérisé en ce que** l'enveloppe (13) réalisée à partir d'une coupe de tissu ou de toile (35) entourant la chambre de dilatation (14) ainsi que la section de chambre et/ou de flexible (15) la reliant à la chambre de compression (16) est assemblée par couture le long de deux zones périphériques (42, 43) de façon à ce qu'il reste au total au moins une ouverture entre les deux coutures (42, 43) et/ou entre ces coutures (42, 43) et une section (36) formée par pliage de la coupe (35).

4. Dispositif (1) selon l'une des revendications 1 à 3, **caractérisé en ce qu'**une ouverture de la coupe de tissu ou de toile (35) cousue en une enveloppe (13) sert à passer la section de chambre et/ou de flexible (15) reliant la chambre de dilatation (14) à la chambre de compression (16).

5. Dispositif (1) selon l'une des revendications précédentes, **caractérisé en ce que** deux ouvertures de la coupe de tissu ou de toile (35) cousue en une enveloppe (13)servent à passer deux extrémités de la bande de fixation (10) entourant la cheville, comprimant la chambre de dilatation (14) contre la malléole interne, **en ce que** ces ouvertures peuvent également être fermées par les coutures correspondantes (42, 43) en cousant l'enveloppe (13) avec la bande de fixation (10).

6. Dispositif (1) selon l'une des revendications précédentes, **caractérisé en ce qu'**une coupe de tissu ou de toile textile tolérée par la peau, entourant au moins partiellement la chambre de compression (16), est réalisée en tissu éponge.

7. Dispositif (1) selon l'une des revendications précédentes, **caractérisé en ce que** la chambre de dilatation (14) entre la malléole interne et le tendon d'Achille est disposée en contact direct avec le système de veines perforantes local, de façon à ce que le sang de la veine perforante locale soit comprimé dans la veine profondeen cas de compression de la chambre de compression (16), disposée au niveau de la plante du pied par la chambre de dilatation (14) se dilatant disposée au niveau de la malléole interne.

8. Dispositif (1) selon l'une des revendications précédentes, **caractérisé en ce qu'**un corps élastique,par exemple un bloc de mousse souple, est disposé dans la chambre de compression (16).

9. Dispositif (1) selon l'une desrevendications précédentes, **caractérisé en ce que** les soudures (28) entre les deux couches du film plastique (17) sont disposées à des intervalles rapprochés de 5 mm ou moins.

10. Dispositif (1) selon l'une des revendications précédentes, **caractérisé en ce qu'**il est disposé à l'intérieur d'un bas (2).

11. Dispositif (1) selon la revendication 10, **caractérisé en ce qu'**une coupe de tissu ou de toile textile en tissu éponge, tolérée par la peau, entourant au moins partiellement la chambre de compression (16) est cousue avec le bas (2), de préférence avec sa face intérieure, en particulier avec sa section de talon (5).

12. Dispositif (1) selon la revendication 10 ou 11, **caractérisé en ce que** des zones de la semelle du bas (2) recevant le dispositif (1) selon l'invention sont constituées de tissu éponge.

13. Dispositif (1) selon l'une des revendications 10 à 12, **caractérisé en ce que** des zones du bas (2) recevant le dispositif (1) selon l'invention sont réalisées avec un autre point de tricot que les autres, en particulier afin que le bas (2) présente partiellement une plus haute élasticité.

14. Dispositif (1) selon l'une des revendications 10 à 13, **caractérisé en ce que** le bas (2) recevant le dispositif (1) selon l'invention comportedans la zone en dessous de son ouverture une manchette(8) qui est rabattable par le biais d'une bande de fixation (10) enroulée autour de la cheville.

15. Dispositif (1) selon l'une des revendications 10 à 14, **caractérisé en ce que** le bas (2) recevant le dispositif (1) selon l'invention présente une texture douce dans la zone entre son ouverture et une manchette (8) rapportée, en particulier qu'il est tricoté en grosses mailles.
